# EUROPÄISCHE PATENTSCHRIFT

(11) **EP 0 961 767 B1**
(45) Veröffentlichungstag und Bekanntmachung des Hinweises auf die Patenterteilung: **10.04.2002**
(21) Anmeldenummer: 98908025.4
(22) Anmeldetag: 30.01.1998
(51) Int. Cl.: C07C 209/84

(54) **VERFAHREN ZUR GEWINNUNG VON HEXAMETHYLENDIAMIN AUS HEXAMETHYLENDIAMIN ENTHALTENDEN MISCHUNGEN**
METHOD FOR OBTAINING HEXAMETHYLENE DIAMINE FROM MIXTURES CONTAINING HEXAMETHYLENE DIAMINE
PROCEDE DE PREPARATION DE DIAMINE D'HEXAMETHYLENE A PARTIR DE MELANGES CONTENANT DE LA DIAMINE D'HEXAMETHYLENE

(30) Priorität: 07.02.1997 DE 19704612
(43) Veröffentlichungstag der Anmeldung: 08.12.1999
(73) Patentinhaber: BASF AKTIENGESELLSCHAFT, 67056 Ludwigshafen (DE)
(72) Erfinder: BASSLER, Peter, D-68519 Viernheim (DE); FISCHER, Rolf, D-69121 Heidelberg (DE); LUYKEN, Hermann, D-67069 Ludwigshafen (DE); REHFINGER, Alwin, D-67112 Mutterstadt (DE); VOIT, Guido, D-67251 Freinsheim (DE)
(86) Internationale Anmeldenummer: EP9800506
(87) Internationale Veröffentlichungsnummer: WO9834903

(56) Entgegenhaltungen:
- DE-A- 19 500 222
- DE-B- 1 204 681
- CHEMICAL ABSTRACTS, vol. 76, no. 5, 31.Januar 1972 Columbus, Ohio, US; abstract no. 24676, POPOV, D. M. ET AL: "Separating the products of adiponitrile hydrogenation" XP002066045 & SU 316 329 A (POPOV, D. M.;MARTYNENKO, R. L.; SHEKHTMAN, E. SH.; MAEVSKII, V. E.; DO)

## Beschreibung

Die vorliegende Erfindung betrifft ein Verfahren zur Gewinnung von Hexamethylendiamin (I) aus einer Mischung (II) enthaltend
(I) Hexamethylendiamin,
(III) Hexamethylenimin,
(IV) eine Verbindung ausgewählt aus der Gruppe bestehend aus 2-Aminomethylcyclopentylamin und 1,2-Diaminocyclohexan,
(v) ein Imin
(VI) Adipodinitril und 6-Aminocapronsäurenitril,
wobei man
(a) eine Mischung (II) einer Destillation in einer Kolonne bei einem Druckabfall vom Sumpf zum Kopf von 1 bis 500 mbar, einem Druck im Sumpf im Bereich von 1 bis 500 mbar, einem Druck im Kopf im Bereich von 1 bis 500 mbar und einer Sumpftemperatur von 100 bis 300° unter Erhalt
   (a1) einer leichtsiedenden Fraktion, welche im wesentlichen (III) enthält, als Kopfprodukt
   (a2) einer mittelsiedenden Fraktion (VII) enthaltend (I), (IV), (V) als Seitenabzug und
   (a3) einer hochsiedenden Fraktion, welche (V) und (VI) enthält, als Sumpfprodukt
   unterwirft,
(b) eine Mischung (VII) einer Destillation unter Erhalt
   (b1) eines Kopfproduktes, welches im wesentlichen (IV) enthält
      und
   (b2) einer Mischung (VIII) enthaltend (I) und (v) als Sumpfprodukt
   unterwirft und
(c) eine Mischung (VIII) einer Destillation unter Erhalt
   (c1) von (I) als Kopfprodukt und
   (c2) eines Sumpfproduktes, welches den wesentlichen Teil von (V) enthält,
   unterwirft.

Es ist bekannt, beispielsweise aus: K. Weissermel, H.-J. Arpe, Industrielle Organische Chemie, 4. Auflage, Seite 270, VCH-Verlagsgesellschaft, Adipodinitril in Gegenwart von Eisen-, Cobaltoder Nickelkatalysatoren vollständig zu dem Faservorprodukt Hexamethylendiamin zu hydrieren. Infolge des quantitativen Umsatzes des Adipodinitrils zu Hexamethylendiamin enthält der Hydrieraustrag praktisch kein Adipodinitril und kein 6-Aminocapronitril.

Die Reinigung des Hexamethylendiamins auf Faserqualität kann in an sich bekannter Weise, beispielsweise gemäß GB-A-731 819 durch Destillation oder gemäß US-A-4,282,381 durch Kristallisation, erfolgen.

Es ist weiterhin bekannt, Adipodinitril partiell zu Gemischen aus den beiden Faservorprodukten 6-Aminocapronitril und Hexamethylendiamin sowie nicht umgesetztem Adipodinitril, beispielsweise nach US-A-4,601,8591, US-A-2,762,835, US-A-2,208,598, DE-A 848 654, DE-A-44 46 893, DE-A-954 416, DE-A-42 35 466, WO 92/21650, DE-A-19 500 222 oder der Deutschen Anmeldung 19 548 289.1 in Gegenwart von Nickel-, Cobalt-, Eisen-, Ruthenium- oder Rhodiumhaltigen Katalysatoren zu hydrieren.

6-Aminocapronitril kann zu Caprolactam cyclisiert oder direkt zu Nylon 6 polymerisiert werden.

Bei der partiellen Hydrierung fallen Nebenprodukte an, die nur schwierig von Hexamethylendiamin abgetrennt werden können, wie Hexamethylenimin, 1,2-Diaminocyclohexan und 2-Aminomethylcyclopentylamin. Vor allem Tetrahydroazepin und die beiden cyclischen Diamine mindern, beispielsweise durch Farbgebung, die Qualität von aus derart verunreinigtem Hexamethylendiamin hergestelltem Nylon 6.6. Daher müssen die genannten Verunreinigungen bis auf Restgehalte von wenigen ppm aus dem Hexamethylendiamin abgetrennt werden.

Die für die Reinigung von durch vollständige Hydrierung von Adipodinitril hergestelltem Hexamethylendiamin bekannten Verfahren lassen sich auf das durch partielle Hydrierung von Adipodinitril erhaltene Reaktionsgemisch nicht übertragen wegen der höheren Gehalte an diesen Nebenprodukten, der abweichenden Mengenverhältnisse der Nebenprodukte sowie der Anwesenheit von 6-Aminocapronsäurenitril und Adipodinitril.

DE-B-12 04 681 offenbart ein Verfahren zur Gewinnung von Hexamethylendiamin mittels eines Destillationsverfahrens unter Verwendung von vier Kolonnen und eines Stau- oder Verweilzeitbehälters.

Aufgabe der vorliegenden Erfindung war es daher, ein Verfahren zur Verfügung zu stellen, das die Gewinnung von Hexamethylendiamin aus einem durch partielle Hydrierung von Adipodinitril zu einem Hexamethylendiamin, 6-Aminocapronsäurenitril und Adipodinitril enthaltenden Gemisch auf technisch einfache und wirtschaftliche Weise ermöglicht.

Demgemäß wurde das eingangs beschriebene Verfahren gefunden.

Mischungen (II) können in an sich bekannter Weise erhalten werden durch partielle Hydrierung von Adipodinitril , beispielsweise nach einem Verfahren gemäß EP-A-161 419, EP-A-77 911, US-A-4,389,348, US-A-4,601,859, WO 93/1207, DE-A 42 35 466, US-A-2,762,835, US-A-2,208,598, DE-A 848 654, DE-A-44 46 893, DE-A-954 416, DE-A-42 35 466, WO 92/21650, DE-A 19 500 222 und der Deutschen Anmeldung 19 548 289.1, indem man im allgemeinen die Hydrierung in Gegenwart von Nickel-, Cobalt-, Eisen-, Rhodium- oder Ruthenium-haltigen Katalysatoren durchführt. Dabei können die Katalysatoren als Trägerkatalysatoren oder als Vollkatalysatroen verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht.

Wurden bei der Hydrierung flüssige Verdünnungsmittel ,wie Ammoniak, eingesetzt, so können diese in an sich bekannter Weise, beispielsweise gemäß DE-A-19 500 222, entfernt werden.

Die Mischungen (II) enthalten im allgemeinen

Hexamethylendiamin (I), insbesondere in Mengen von 5 bis 90 Gew.-% bezogen auf (II),

Hexamethylenimin (III), insbesondere in Mengen von 0.1 bis 10 Gew.-% bezogen auf (II),
eine Verbindung (IV) ausgewählt aus der Gruppe bestehend aus 2-Aminomethylcyclopentylamin und 1,2-Diaminocyclohexan oder deren Gemische, insbesondere in Mengen von 5 ppm bis 5 Gew.-% bezogen auf (II),
ein Imin (V) oder Gemische solcher Imine, wie Tetrahydroazepin, wobei die Imine in einzelnen Verbindungen oder als Addukte, insbesondere mit Aminen wie Hexamethylendiamin oder 6-Aminocapronsäurenitril, vorliegen können und im Sinne der vorliegenden Erfindung solche Addukte als Imine (V) bezeichnet werden, insbesondere in Mengen von 5 bis 10 000 ppm bezogen auf (II) Adipodinitril und 6-Aminocapronsäurenitril (VI), wobei der Gehalt an 6-Aminocapronsäurenitril 5 bis 90 Gew.-% bezogen auf (II) und der Gehalt an Adipodinitril 5 bis 90 Gew.-% bezogen auf (II) betragen kann.

Erfindungsgemäß unterwirft man die Mischung (I) einer Destillation (a).

Bei dieser Destillation erhält man als leichtsiedende Fraktion im wesentlichen Hexamethylenimin (III). Diese leichtsiedende Fraktion (a1) kann weitere Verbindungen enthalten, wie Reste des bei der Hydrierung eingesetzten flüssigen Verdünnungsmittels, beispielsweise Ammoniak, oder Reste des bei der Hydrierung als Nebenprodukt angefallenen Wassers.

Als mittelsiedende Fraktion (a2) erhält man eine Mischung (VII) enthaltend im wesentlichen Hexamethylendiamin (I), eine Verbindung (IV) und eine Verbindung (IVb) wobei der Gehalt an Hexamethylendiamin in der Mischung (VII) vorzugsweise von 80 bis 100 Gew.-% bezogen auf (VII), der Gehalt an Verbindung (IV) in der Mischung (VII) vorzugsweise von 5 ppm bis 0.5 Gew.-% bezogen auf (VII) und der Gehalt an Verbindung (IVb) in der Mischung (VII) vorzugsweise von 1 bis 10 000 ppm bezogen auf (VII) beträgt.

Als hochsiedende Fraktion (a3) erhält man ein im wesentlichen Adipodinitril und 6-Aminocapronsäurenitril enthaltendes Gemisch (VI), aus dem Adipodinitril und 6-Aminocapronsäurenitril vorzugsweise destillativ gewonnen werden können. Das Adipodinitril kann vorteilhaft wieder in die beschriebene partielle Hydrierung zurückgeführt werden.

Für die Destillation kommen hierfür übliche Destillationskolonnen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Erfindungsgemäß sind Destillationsapparaturen, die vom Sumpf zum Kopf einen Druckabfall von 1 bis 500 mbar, vorzugsweise 5 bis 50 mbar aufweisen, wobei der Druck im Sumpf im Bereich von 1 bis 500 mbar und im Kopf im Bereich von 1 bis 500 mbar liegt. Hierdurch ergeben sich Sumpftemperaturen von 100 bis 300, insbesondere 150 bis 250°C.

Erfindungsgemäß führt man die Destillation in einer Kolonne durch, so daß man (a1) als Kopfprodukt, (a2) als Seitenabzug und (a3) als Sumpfprodukt erhält.

Vorteilhafterweise weist das Destillationsgemisch auf mindestens einer, vorzugsweise 1 bis 15, besonders bevorzugt 1 bis 7, insbesondere 1, 2 oder 3 Ebenen der Destillationskolonne eine durchschnittliche mittler Verweilzeit von mindestens 5 Minuten, vorzugsweise mindestens 15 Minuten, insbesondere mindestens 45 Minuten auf.

Vorzugsweise entnimmt man der Destillationskolonne das Destillationsgemisch auf mindestens einer Ebene, leitet es durch einen Verweilzeitbehälter und führt es der Destillationskolonne wieder zu. Die Rückführung kann dabei auf die Abzugsebene oder auf eine Ebene oberhalb oder unterhalb der Abzugsebene erfolgen.

Vorteilhaft kann der Rücklauf der Destillationskolonne zunächst durch einen Verweilzeitbehälter und anschließend in die Destillationskolonne zurückgeführt werden.

Das Entnehmen von Destillationsflüssigkeit aus der Kolonne, Leiten durch den Verweilzeitbehälter, Zurückführen in die Destillationskolonne und gegebenenfalls Umwälzung der Flüssigkeit im Verweilzeitbehälter kann mit an sich bekannten Apparaturen, wie Pumpen, erfolgen, wobei die Zurückführung auf die Abzugsebene der Destillationskolonne, insbesondere bei einer Bodenkolonne, oder auf eine oberhalb, insbesondere bei einer Packungskolonne, oder unterhalb der Abzugsebene liegende Ebene erfolgen kann.

Die Destillation der Mischung (II) kann vorteilhaft unter Zugabe einer gegenüber den Komponenten der Mischung unter den Destillationsbedingungen inerten Verbindung (IX), deren Siedepunkt unter den Destillationsbedingungen über dem Siedepunkt der mittelsiedenden Fraktion (a2) liegt, erfolgen.

Als Verbindungen (IX) kommen Verbindungen aus der Gruppe der Aromaten, der Aliphaten, wie acyclische und cyclische Aliphaten, und aliphatisch-aromatische Verbindungen in Betracht. Diese Verbindungen können Substituenten tragen, wie eine Alkyl-, Aryl-Cycolalkyl-, Aralkyl-, Ester-, Amid-, Nitril- oder Amino-Gruppe, vorzugsweise Nitril- oder Amino-Gruppe, oder mehrere gleiche oder unterschiedliche solcher Gruppen.

Die Verbindung (IX) kann aus einer Verbindung oder Gemischen solcher Verbindungen bestehen.

Vorteilhaft sind solche Verbindungen (IX), die sich auf einfache Weise, wie durch Hydrierung beispielsweise mit einem molekularen Wasserstoff enthaltenden Gas in Gegenwart eines Katalysators, zu Hexamethylendiamin oder 6-Aminocapronsäurenitril umsetzen lassen.

Die bei dieser Umsetzung erhaltenen Produkte können in das erfindungsgemäße Verfahren vorteilhaft erneut eingesetzt werden. Nach der Destillation erhält man, vorzugsweise als Sumpfprodukt, eine Mischung enthaltend eine Verbindung (IX).

Enthält die Mischung zusätzlich Hexamethylendiamin (I), so kann vorteilhaft die Sumpftemperatur bei der Destillatiom herabgesetzt werden.

Aus der Mischung kann in an sich bekannter Weise, beispielsweise durch physikalische Verfahren, wie Destillation oder Extraktion, oder chemische Verfahren, wie Chemisorption oder Hydrierung, die Verbindung (IX) zurückgewonnen werden.

Diese aus der Mischung erhaltene Verbindung (IX) kann vorteilhaft in die
Schritte (a) oder (c) oder beide Schritte zurückgeführt werden. Die Differenz der Siedepunkte zwischen dem der mittelsiedenden Fraktion (a2) und der Verbindung (IX) sollte unter desn Destillationsbedingungen 1 bis 200°C vorzugsweise 5 bis 100°C betragen. Als besonders vorteilhaft hat sich der Einsatz von Adipodinitril oder 6-Aminocapronsäurenitril oder deren Gemische erwiesen.

Die Verbindung (IX) kann zu der Mischung (II) vor oder während der Destillation zugegeben werden.

Die Zugabe der Verbindung (IX) zu der Mischung (II) vor der Destillation kann in an sich bekannter Weise in üblichen Mischapparturen erfolgen.

Die Zugabe der Verbindung (IX) zu der Mischung (II) während der Destillation kann durch Einspeisen der Verbindung (IX) in die Destillationsapparatur vorzugsweise in den Sumpf erfolgen.

Die Destillation der Mischung (II) kann vorteilhaft in Gegenwart von Kohlendioxid erfolgen.

Kohlendioxid kann dem Destillationsgemisch vor oder vorzugsweise während der Destillation in Form einer Verbindung, die unter den Destillationsbedingungen Kohlendioxid freisetzt, wie Ammoniumcarbonat, Ammoniumcarbamat oder Harnstoff oder Gemische solcher Verbindungen, wobei diese Verbindungen in reiner Form oder in einem flüssigen Verdünnungsmittel, wie einem oder mehreren Bestandteilen der Mischung (II), zugegeben werden können, oder in Form von festem, flüssigem oder vorzugsweise gasförmigem Kohlendioxid, beispielsweise in Form eines Kohlendioxid enthaltenden Gases oder insbesondere in Form von reinem, nur die üblichen Verunreinigungen enthaltendem gasförmigem Kohlendioxid, erfolgen. Der Kohlendioxid-Gehalt im Destillationsgemisch sollte 0.1 bis 1 000 mol Kohlendioxid pro mol Iminfunktion der Imine, wie Tetrahydroazepin, betragen.

Erfindungsgemäß unterwirft man die Mischung (VII) einer Destillation (b).

Bei dieser Destillation erhält man als Kopfprodukt (b1) eine Verbindung (IV) enthaltend im wesentlichen 2-Aminomethylcyclopentylamin, 1,2-Diaminocyclohexan oder deren Gemische. Zudem kann das Kopfprodukt Hexamethylenimin oder Hexamethylendiamin oder deren Gemische enthalten.

Als Sumpfprodukt (b2) der Destillationskolonne erhält man eine Mischung (VIII) enthaltend im wesentlichen Hexamethylendiamin (I) und ein Imin (V), wobei der Gehalt an Hexamethylendiamin in der Mischung (VIII) vorzugsweise von 50 bis 100 Gew.-% bezogen auf (VIII) beträgt.

Für die Destillation kommen hierfür übliche Destillationskolonnen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Bevorzugt sind Destillationsapparturen, die vom Sumpf zum Kopf einen Druckabfall von 0 bis 200 mbar, vorzugsweise 0 bis 50 mbar aufweisen, wobei vorteilhaft der Druck im Sumpf im Bereich von 3 bis 300 mbar, insbesondere 1 bis 200 mbar, und im Kopf im Bereich von 1 bis 300 mbar, insbesondere 1 bis 200 mbar, liegen sollte. Hierdurch ergeben sich Sumpftemperaturen von 100 bis 300, insbesondere 150 bis 250°C.

Die Destillation kann man in mehreren, wie 2, 3 oder mehr Kolonnen, vorteilhaft einer einzigen Kolonne durchführen.

Besonders geeignet sind Destillationskolonnen, die einen geringen Druckverlust, vorzugsweise höchstens 1 mbar, insbesondere 0,3 mbar pro theoretischer Trennstufe aufweisen.

Insbesondere geeignet sind Packungskolonnen, vorzugsweise mit geordneten Füllkörpern wie Metallblechpackungen, insbesondere Drahtgewebepackungen.

Vorteilhaft kann dem Destillationsgemisch Wasser zugegeben werden, wobei Wassergehalte von 0.001 bis 10., insbesondere 0.01 bis 5 Gew.-% Wasser bezogen auf das Destillationsgemisch bevorzugt sind.

Das Wasser kann dabei dem Gemisch vor der Destillation oder während der Destillation, beispielsweise in die Kolonne in den unteren Bereich, zugegeben werden.

Erfindungsgemäß unterwirft man die Mischung (VIII) einer Destillation (c).

Bei dieser Destillation erhält man als Kopfprodukt (c1) Hexamethylendiamin (I), vorteilhaft in für die Faserherstellung geeigneten Reinheit.

Als Sumpfprodukt (c2) erhält man wesentliche Teile von (V) sowie andere Verbindungen, die einen höheren Siedepunkt als Hexamethylendiamin aufweisen.

Für die Destillation kommen hierfür übliche Destillationskolonnen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Bevorzugt sind Destillationsapparturen, die vom Sumpf zum Kopf einen Druckabfall von 1 bis 1000 mbar, vorzugsweise 1 bis 500 mbar aufweisen, wobei vorteilhaft der Druck im Sumpf im Bereich von 10 bis 900 mbar und im Kopf im Bereich von 50 bis 500 mbar liegen sollte. Hierdurch ergeben sich Sumpftemperaturen von 100 bis 300, insbesondere 150 bis 250°C.

Die Destillation kann man in mehreren, wie 2 oder 3 Kolonnen, vorteilhaft einer einzigen Kolonne durchführen.

Vorteilhafterweise weist das Destillationsgemisch auf mindestens einer, vorzugsweise 1 bis 15, besonders bevorzugt 1 bis 7, insbesondere 1, 2 oder 3 Ebenen der Destillationskolonne eine durchschnittliche mittler Verweilzeit von mindestens 5 Minuten, vorzugsweise mindestens 15 Minuten, insbesondere mindestens 45 Minuten auf.

Vorzugsweise entnimmt man der Destillationskolonne das Destillai tionsgemisch auf mindestens einer Ebene, leitet es durch einen Verweilzeitbehälter und führt es der Destillationskolonne wieder zu. Die Rückführung kann dabei auf die Abzugsebene oder auf eine Ebene oberhalb oder unterhalb der Abzugsebene erfolgen. Vorteilhaft kann der Rücklauf der Destillationskolonne zunächst durch einen Verweilzeitbehälter und anschließend in die Destillationskolonne zurückgeführt werden.

Das Entnehmen von Destillationsflüssigkeit aus der Kolonne, Leiten durch den Verweilzeitbehälter, Zurückführen in die Destillationskolonne und gegebenenfalls Umwälzung der Flüssigkeit im Verweilzeitbehälter kann mit an sich bekannten Apparaturen, wie Pumpen, erfolgen, wobei die Zurückführung auf die Abzugsebene der Destillationskolonne, insbesondere bie einer Bodenkolonne, oder auf eine oberhalb, insbesondere bei einer Packungskolonne, oder unterhalb der Abzugsebene liegende Ebene erfolgen kann.
Die Destillation der Mischung (VIII) kann vorteilhaft unter Zugabe einer gegenüber den Komponenten der Mischung unter den Destillationsbedingungen inerten Verbindung (X), deren Siedepunkt unter den Destillationsbedingungen über dem Siedepunkt von Hexamethylendiamin (I) liegt, erfolgen.

Als Verbindungen (X) kommen Verbindungen aus der Gruppe der Aromaten, der Aliphaten, wie acyclische und cyclische Aliphaten, und aliphatisch-aromatische Verbindungen in Betracht. Diese Verbindungen können Substituenten tragen, wie eine Alkyl-, Aryl-Cycolalkyl-, Aralkyl-, Ester-, Amid-, Nitril- oder Amino-Gruppe, vorzugsweise Nitril- oder Amino-Gruppe, oder mehrere gleiche oder unterschiedliche solcher Gruppen.

Die Verbindung (X) kann aus einer Verbindung oder Gemischen solcher Verbindungen bestehen.

Vorteilhaft sind solche Verbindungen (X), die sich auf einfache Weise, wie durch Hydrierung beispielsweise mit einem molekularen Wasserstoff enthaltenden Gas in Gegenwart eines Katalysators, zu Hexamethylendiamin oder 6-Aminocapronsäurenitril umsetzen lassen.

Die bei dieser Umsetzung erhaltenen Produkte können in das erfindungsgemäße Verfahren vorteilhaft erneut eingesetzt werden. Nach der Destillation erhält man, vorzugsweise als Sumpfprodukt, eine Mischung enthaltend eine Verbindung (X).

Enthält die Mischung zusätzlich Hexamethylendiamin (I), so kann vorteilhaft die Sumpftemperatur bei der Destillation herabgesetzt werden.

Aus der Mischung kann in an sich bekannter Weise, beispielsweise durch physikalische Verfahren, wie Destillation oder Extraktion, oder chemische Verfahren, wie Chemisorption oder Hydrierung, die Verbindung (X) zurückgewonnen werden.

Diese aus der Mischung erhaltene Verbindung (X) kann vorteilhaft in die Schritte (a) oder (c) oder beide Schritte zurückgeführt werden.

Die Differenz der Siedepunkte zwischen Hexamethylendiamin (I) und der Verbindung (X) sollte unter den Destillationsbedingungen 1 bis 200°C, vorzugsweise 5 bis 100°C betragen.

Als besonders vorteilhaft hat sich der Einsatz von Adipodinitril, 6-Aminocapronsäurenitril oder deren Gemische erwiesen.

Die Verbindung (X) kann zu der Mischung (VIII) vor oder während der Destillation zugegeben werden.

Die Zugabe der Verbindung (X) zu der Mischung (VIII) vor der Destillation kann in an sich bekannter Weise in üblichen Mischapparturen erfolgen.

Die Zugabe der Verbindung (X) zu der Mischung (VIII) während der Destillation kann durch Einspeisen der Verbindung (X) in die Destillationsapparatur vorzugsweise in den Sumpf erfolgen.

Die Destillation der Mischung (VIII) kann vorteilhaft in Gegenwart von Kohlendioxid erfolgen.

Kohlendioxid kann dem Destillationsgemisch vor oder vorzugsweise während der Destillation in Form einer Verbindung, die unter den Destillationsbedingungen Kohlendioxid freisetzt, wie Ammoniumcarbonat, Ammoniumcarbamat oder Harnstoff oder Gemische solcher Verbindungen, wobei diese Verbindungen in reiner Form oder in einem flüssigen Verdünnungsmittel, wie einem oder mehreren Bestandteilen der Mischung (VIII), zugegeben werden können, oder in Form von festem, flüssigem oder vorzugsweise gasförmigem Kohlendioxid, beispielsweise in Form eines Kohlendioxid enthaltenden Gases oder insbesondere in Form von reinem, nur die üblichen Verunreinigungen enthaltendem gasförmigem Kohlendioxid, erfolgen.

Der Kohlendioxid-Gehalt im Destillationsgemisch sollte 0,01 bis 1000 mol Kohlendioxid pro mol Iminfunktion der Imine, wie Tetrahydroazepin, betragen.

## Patentansprüche

1. Verfahren zur Gewinnung von Hexamethylendiamin (I) aus einer Mischung (II) enthaltend
(I) Hexamethylendiamin,
(III) Hexamethylenimin,
(IV) eine Verbindung ausgewählt aus der Gruppe bestehend aus 2-Aminomethylcyclopentylamin und 1,2-Diaminocyclohexan,
(V) ein Imin
(VI) Adipodinitril und 6-Aminocapronsäurenitril,
wobei man
(a) eine Mischung (II) einer Destillation in einer Kolonne bei einem Druckabfall vom Sumpf zum Kopf von bis 500 mbar, einem Druck im Sumpf im Bereich von 1 bis 500 mbar, einem Druck im Kopf im Bereich von 1 bis 500 mbar und einer Sumpftemperatur von 100 bis 300°C unter Erhalt
(a1) einer leichtsiedenden Fraktion, welche im wesentlichen (III) enthält, als Kopfprodukt
(a2) einer mittelsiedenden Fraktion (VII) enthaltend (I), (IV), (V) als Seitenabzug und
(a3) einer hochsiedenden Fraktion, welche (V) und (VI) enthält, als Sumpfprodukt
unterwirft,
(b) eine Mischung (VII) einer Destillation unter Erhalt
(b1) eines Kopfproduktes, welches im wesentlichen (IV) enthält und
(b2) einer Mischung (VIII) enthaltend (I) und (V) als Sumpfprodukt
unterwirft und
(c) eine Mischung (VIII) einer Destillation unter Erhalt
(c1) von (I) als Kopfprodukt und
(c2) eines Sumprproduktes, welches den wesentlichen Teil von (V) enthält,
unterwirft.

2. Verfahren nach Anspruch 1, wobei in Schritt (a) die mittlere durchschnittliche Verweilzeit der Destillationsmischung in der Destillationsapparatur mindestens 5 Minuten beträgt.

3. Verfahren nach Anspruch 1 oder 2, wobei in Schritt (c) die mittlere durchschnittliche Verweilzeit der Destillationsmischung in der Destillationsapparatur mindestens 5 Minuten beträgt.

4. Verfahren nach den Ansprüchen 1 bis 3, wobei man in Schritt (a) bei der Destillation Kohlendioxid zugibt.

5. Verfahren nach den Ansprüchen 1 bis 4, wobei man in Schritt (c) bei der Destillation Kohlendioxid zugibt.

6. Verfahren nach den Ansprüchen 1 bis 5, wobei man in Schritt (a) dem Destillationsgemisch eine gegenüber den Komponenten der Mischung unter den Destillationsbedingungen inerte Verbindung (IX) mit einem Siedepunkt über dem Siedepunkt des Hexamethylendiamin (I) zugibt.

7. Verfahren nach den Ansprüchen 1 bis 6, wobei man in Schritt (c) dem Destillationsgemisch oder der Destillationsapparatur eine Verbindung (X) mit einem Siedepunkt über dem Siedpunkt der Mischung (VII) zugibt.

8. Verfahren nach Anspruch 7, wobei die Verbindung (X) eine Verbindung (VI) enthält.

9. Verfahren nach den Ansprüchen 7 oder 8, wobei man als Verbindung (X) Adipodinitril, 6-Aminocapronsäurenitril oder deren Gemische zugibt.

10. Verfahren nach den Ansprüchen 7 bis 9, wobei die Verbindung (X) Sumpfprodukt aus Schritt (a3) enthält.

11. Verfahren nach den Ansprüchen 1 bis 10, wobei man in Schritt (b) dem Destillationsgemisch Wasser zugibt.

## Claims

1. A process for recovering hexamethylenediamine (I) from a mixture (II) comprising
(I) hexamethylenediamine,
(III) hexamethyleneimine,
(IV) a compound selected from the group consisting of 2-aminomethylcyclopentylamine and 1,2-diaminocyclohexane,
(V) an imine,
(VI) adiponitrile and 6-aminocapronitrile,
which comprises distilling
(a) a mixture (II) in a column at a pressure drop from the bottom to the top of from 1 to 500 mbar, a pressure in the bottom within the range from 1 to 500 mbar, a pressure at the top within the range from 1 to 500 mbar and a bottom temperature of from 100 to 300°C to obtain
(a1) a low boiling fraction comprising essentially (III) as overhead product,
(a2)a medium boiling fraction (VII) comprising (I), (IV) and (V) as sidestream takeoff, and
(a3)a high boiling fraction comprising (V) and (VI) as bottom product,
(b) a mixture (VII) to obtain
(b1) an overhead product comprising essentially (IV), and
(b2)a mixture (VIII) comprising (I) and (V) as bottom product, and
(c) a mixture (VIII) to obtain
(c1) (I) as overhead product, and
(c2)a bottom product comprising the essential portion-of (V).

2. A process as claimed in claim 1, wherein the mean average residence time of the distillation mixture in the distillation apparatus is at least 5 minutes in step (a).

3. A process as claimed in claim 1 or 2, wherein the mean average residence time of the distillation mixture in the distillation apparatus is at least 5 minutes in step (c).

4. A process as claimed in any of claims 1 to 3, wherein carbon dioxide is added to the distillation in step (a).

5. A process as claimed in any of claims 1 to 4, wherein carbon dioxide is added to the distillation in step (c).

6. A process as claimed in any of claims 1 to 5, wherein a compound (IX) which is inert toward the components of the mixture under the distillation conditions and has a boiling point above the boiling point of hexamethylenediamine (I) is added to the distillation mixture in step (a).

7. A process as claimed in any of claims 1 to 6, wherein a compound (X) having a boiling point above the boiling point of the mixture (VII) is added to the distillation mixture or apparatus in step (c).

8. A process as claimed in claim 7, wherein said compound (X) comprises a compound (VI).

9. A process as claimed in claim 7 or 8, wherein compound (X) is adiponitrile, 6-aminocapronitrile or a mixture thereof.

10. A process as claimed in any of claims 7 to 9, wherein said compound (X) comprises bottom product from step (a3).

11. A process as claimed in any of claims 1 to 10, wherein water is added to the distillation mixture in step (b).

## Revendications

1. Procédé de préparation d'hexaméthylènediamine (I) à partir d'un mélange contenant
(I) de l'hexaméthylènediamine,
(II) de l'hexaméthylène-imine,
(III) un composé choisi dans le groupe formé par la 2-aminométhylcyclopentylamine et le 1,2-diaminocyclohexane,
(IV) une imine,
(V) de l'adiponitrile et du 6-aminocapronitrile,
dans lequel
(a) on soumet un mélange (II) à une distillation dans une colonne avec une perte de charge du fond de colonne à la tête de 1 à 500 mbar, une pression de fond de colonne de l'ordre de 1 à 500 mbar, une pression en tête de colonne de l'ordre de 1 à 500 mbar, et une température de fond de colonne de 100 à 300°C, avec obtention
(a1) d'une fraction à bas point d'ébullition, contenant essentiellement (III), comme fraction de tête,
(a2) d'une fraction à point d'ébullition moyen (VII), contenant (I), (IV), (V) comme soutirage latéral et
(a3) une fraction à haut point d'ébullition, contenant (V) et (VI), comme fraction de queue;
(b) on soumet un mélange (VII) à une distillation avec obtention
(b1) d'une fraction de tête, contenant essentiellement (IV), et
(b2) d'un mélange (VIII) contenant (I) et (V) comme fraction de queue, et
(c) on soumet un mélange (VIII) à une distillation avec obtention
(c1) de (I) comme fraction de tête et
(c2) d'une fraction de queue contenant une partie substantielle de (V).

2. Procédé selon la revendication 1, où dans l'étape (a) le temps de séjour moyen du mélange de distillation dans l'appareillage de distillation est d'au moins 5 minutes.

3. Procédé selon la revendication 1 ou 2, où dans l'étape (c) le temps de séjour moyen du mélange de distillation dans l'appareillage de distillation est d'au moins 5 minutes.

4. Procédé selon les revendications 1 à 3, où dans l'étape (a) on introduit du dioxyde de carbone pendant la distillation.

5. Procédé selon les revendications 1 à 4, où dans l'étape (c) on introduit du dioxyde de carbone pendant la distillation.

6. Procédé selon les revendications 1 à 5, où dans l'étape (a) on ajoute au mélange de distillation un composé (IX) inerte vis-à-vis des composants du mélange dans les conditions de la distillation, et d'un point d'ébullition supérieur au point d'ébullition de l'hexaméthylènediamine (I).

7. Procédé selon les revendications 1 à 6, où dans l'étape (c) on ajoute au mélange de distillation ou dans l'appareillage de distillation un composé (X) d'un point d'ébullition supérieur au point d'ébullition du mélange (VII).

8. Procédé selon la revendication 7, où le composé (X) contient un composé (VI).

9. Procédé selon les revendications 7 ou 8, où l'on ajoute en tant que composé (X) de l'adiponitrile, du 6-aminocapronitrile ou leurs mélanges.

10. Procédé selon les revendications 7 à 9, où le composé (X) contient la fraction de queue de l'étape (a3).

11. Procédé selon les revendications 1 à 10, où dans l'étape (b) on ajoute de l'eau au mélange de distillation.
